# EUROPEAN PATENT SPECIFICATION

(11) **EP 2 029 183 B1**
(45) Date of publication and mention of the grant of the patent: **10.04.2019**
(21) Application number: 07725066.0
(22) Date of filing: 10.05.2007
(51) Int. Cl.: A61L 9/20, A61L 9/16, F24F 3/16, B01D 53/04, B01D 53/047

(54) **APPARATUS FOR TREATING, PURIFYING AND RECONDITIONING AIR IN ENCLOSED ENVIRONMENTS WITH HUMAN PRESENCE**
VORRICHTUNG ZUR BEHANDLUNG, REINIGUNG UND REKONDITIONIERUNG VON LUFT IN GESCHLOSSENEN UMGEBUNGEN, IN DENEN MENSCHEN ANWESEND SIND
APPAREIL ET PROCEDE DE TRAITEMENT, DE PURIFICATION ET DE RECONDITIONNEMENT D'AIR DANS DES ENVIRONNEMENTS CLOS AVEC PRESENCE HUMAINE

(30) Priority: 10.05.2006 IT MI20060922
(43) Date of publication of application: 04.03.2009
(73) Proprietor: Finanziaria Unterland S.p.A., 39100 Bolzano (IT)
(72) Inventor: PRUNERI, Marco, 39040 Ora (IT)
(74) Representative: Forattini, Amelia
(86) International application number: PCT/EP2007/004144
(87) International publication number: WO 2007/128584

(56) References cited:
- EP-A1- 0 614 681
- WO-A-97/15791
- DE-A1- 3 422 110
- DE-A1- 4 407 969
- DE-U1-202004 009 106
- JP-A- 2 092 374
- US-A- 3 593 711
- US-A- 5 082 471
- US-A- 5 791 982
- US-A- 5 972 077
- US-A1- 2004 187 680
- US-A1- 2005 163 648

## Description

The present invention relates to an apparatus and a method for treating, purifying and reconditioning air in enclosed environments characterized by the presence of human beings.

In particular, the present invention seeks to deal with the problem constituted by the weakening of immune defenses in human beings caused by continuous and increasing atmospheric pollution caused by fine and ultrafine particles.

It is known from medical studies that the percentages of fine and ultrafine particles suspended in the air which are retained by our body can reach, depending on their size, up to 95% of the amount that is inhaled during normal respiratory activity.

These particles are carried by our circulatory system within our body.

The particles are partly expelled through traditional pathways and are partly deposited in varying percentages and quantities within the various tissues.

Furthermore, it is known from medical studies that these particles weaken the immune defenses of human beings.

This weakening has also been characterized and quantified by means of an index of increased likelihood of contracting various contagious diseases.

This likelihood can increase up to 30% with respect to the situation in which the body has not been subjected to this kind of weakening.

The important role that a filtration system capable of suppressing fine and ultrafine particles in enclosed environments can have in everyday life is therefore evident.

In particular, the effectiveness of such a filtration system increases in terms of reducing the negative influence of weakening caused by particle pollution as the time during which the people to be protected remain in "controlled" environments that are characterized by reduced content of suspended particles increases.

Likewise, if the filtration system is also capable of sterilizing the air and of killing microbes and bacteria, during its filtration activity, if the suppression rate is high and ensures a low presence of bacteria and/or microbes in the air, the probability of contagion is reduced consequently even in environments in which sources of infection which use air and suspended particles as a vehicle for infection are present at the same time.

WO97/15791 A discloses a process for providing oxygen-enriched pure air, which is used to flood premises in a building, an "oxygenarium". The air consumed by the subjects is reprocessed and fed back into the oxygenarium with fresh outside air.

US2004/0187680 A discloses a system for providing a person with a low oxygen (hypoxic) environment for training purposes. Oxygen sensors automatically monitor and control oxygen levels to maintain the "altitude" desired. CO2 levels are monitored and CO2 is eliminated.

JPH2092374A discloses a system for reducing the carbon dioxide inside a room by providing an open air intake stream course and a bypass stream course, with discharge rate controlling means according to the output of a carbon dioxide sensor. The system includes a TSA and the air taken in the open air intake stream course is divided into air passing through the TSA and a stream of air that does not pass into the TSA with the by pass stream course.

The aim of the present invention is to provide an apparatus and a method suitable to contrast effectively the effects of the forms of pollution described above.

An object of the invention is to provide an apparatus and a method capable of also solving the problem of reducing the probability of contagion by bacteria and microbes through the air that we breathe in populated enclosed environments and of assisting immune defenses and the human body in reacting more effectively in case of disease.

This aim and these and other objects, which will become better apparent hereinafter, are achieved by an apparatus for treating, purifying and reconditioning air within enclosed environments with human presence, as claimed in the appended claims.

Further characteristics and advantages will become better apparent from the following detailed description of preferred but not exclusive embodiments of the invention, illustrated by way of non-limiting example in the accompanying drawings, wherein:
Figure 1 is a schematic view of an application of the apparatus according to the invention;
Figure 2 is a schematic view of an embodiment of the filtration module;
Figure 3 is a schematic view of an embodiment of the oxygen enrichment module;
Figure 4 is a schematic view of an embodiment of the carbon dioxide absorption module.

With reference to the cited figures, the method for treating, purifying and reconditioning air within enclosed environments with human presence according to the present invention includes essentially the following steps: filtration of fine and ultrafine particles out of the air in the served space; subsequent sterilization of the filtered air; controlled oxygen enrichment of the air within the enclosed space; and suppression of carbon dioxide produced by human respiration.

The method according to the invention can be performed by means of an apparatus, generally designated by the reference numeral 1, which is shown schematically, in the figures, in its application to an enclosed environment, such as a room 2.

The apparatus includes a filtration module 3, an oxygen enrichment module 4, and a carbon dioxide absorption module 5.

The filtration module 3 includes a centrifugal fan 6 or another type of fan, which is in any case characterized by a head curve which is sufficient to ensure good air circulation through a filter 7 for suppressing fine and ultrafine particles, which is preferably of the EPA type meant to retain the particles.

The air enters the system through an air intake 14 and exits through an air outlet 15 after treatment.

The filtration module includes UV lamps 8, that are adapted to kill the bacteria and/or microbes in output from the module, so as to achieve a reduction of the amount of active bacteria and/or microbes that are present in the air.

An oxygen sensor is arranged downstream of the filter 7 and is suitable to detect the percentage of oxygen that is present in the air that passes through the filtration module. The currently preferred technology for the oxygen sensor is the electrochemical cell, which offers the advantages of having presently an average life of approximately 10 years, of not requiring an external supply, and of having a substantial intrinsic strength and very small dimensions.

A PLC 9 for controlling the operation of the module and of the system in general optimizes the operation of the entire system, in function of the control policies chosen by the operator.

In output to the filter 7 is placed a differential pressure sensor 10 or, as an alternative to the differential pressure sensor, a current sensor 11 detecting the current absorbed by the fan 6.

The signal provided by these two sensors is used by the PLC 9 control of the module in order to allow to assess the state of "clogging" of the filter and consequently in order to allow to report to the user the time when the replacement of the filter will be necessary.

A current sensor 12 for measuring the current absorbed by the UV lamps, or alternatively a sensor for measuring the brightness of the same lamps, allows the control PLC 9 of the module to know and report to the utilizer whether the lamps 8 have to be replaced or continue to operate correctly.

An optional sensor for detecting the particles dispersed in the air, located within the module ahead of the filtration chain of the module, allows the control PLC to also implement machine power-on/off policies which depend on the analysis of the quantity of suspended particles within the room to be served.

There is a system 13 for communication between the filtration module 3 and the module 4 for enriching the atmosphere with oxygen and the module 5 for adsorbing carbon dioxide.

This transmission system 13 can be either of the radio type for wireless transmissions or of the wired type with a field bus. The preferred choice is the wireless one, which privileges simplicity of installation and connection of the three modules of the present invention in a generic context. The wired system is used where, due to various technical problems, such as excessive distance between the modules, particularly strong local radio noise, electrically shielded environments, etc, there is no assurance of good radio link quality.

The oxygen enrichment module 4 includes an oxygen generator 16, the currently preferred manufacturing technology of which is the PSA (acronym of Pressure Swing Adsorber), the active element of which is constituted by zeolites.

The module 4 has a PLC 17 for controlling the oxygen generator and a communication system 18 for communication between the three modules.

The oxygen enrichment module 4 has an oxygen sensor 19, the signal of which is used by the control PLC to manage the operation of the PSA.

Preferably, the PSA generator is soundproofed by means of a soundproofing system 20 of a per se known type.

The oxygen enrichment module 4 has an air intake 21 and a treated air outlet 22.

The carbon dioxide absorption module 5, meant to suppress the carbon dioxide in the air within the environment 2, includes a CO₂ adsorber, designated by the reference numeral 23.

The carbon dioxide adsorber module 5 can be realized for example with TSA technology (acronym of Temperature Swing Adsorber), the active element of which is constituted by zeolites, or with any other equivalent technology.

A PLC 24 for controlling the adsorber cooperates with a communication system 25 for communication between the modules and uses the signal generated by a carbon dioxide sensor 26 to manage the operation of the TSA.

Of course, the carbon dioxide adsorber module 5 has an air intake 27 and a treated air outlet 28.

The construction geometry of the filtration module harmonizes well with the furnishings of rooms of any kind, being it either offices or rooms located within dwellings.

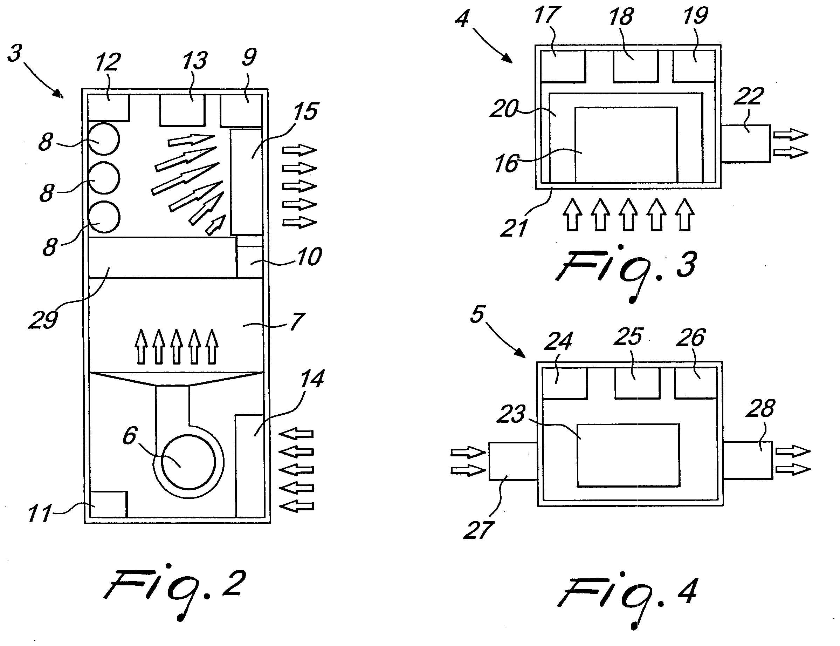

In particular, the filtration unit can be realized as a stand-alone cabinet, similar in shape and size to the fan coils and radiators for heating which are currently present in any room of every public or private building, or can be integrated in climate-control units. In this second case, the filtration chain of the first module described above is completed by the evaporator 29 used to cool and/or heat the air, as can be seen currently in any climate control system, as shown schematically in Figure 2.

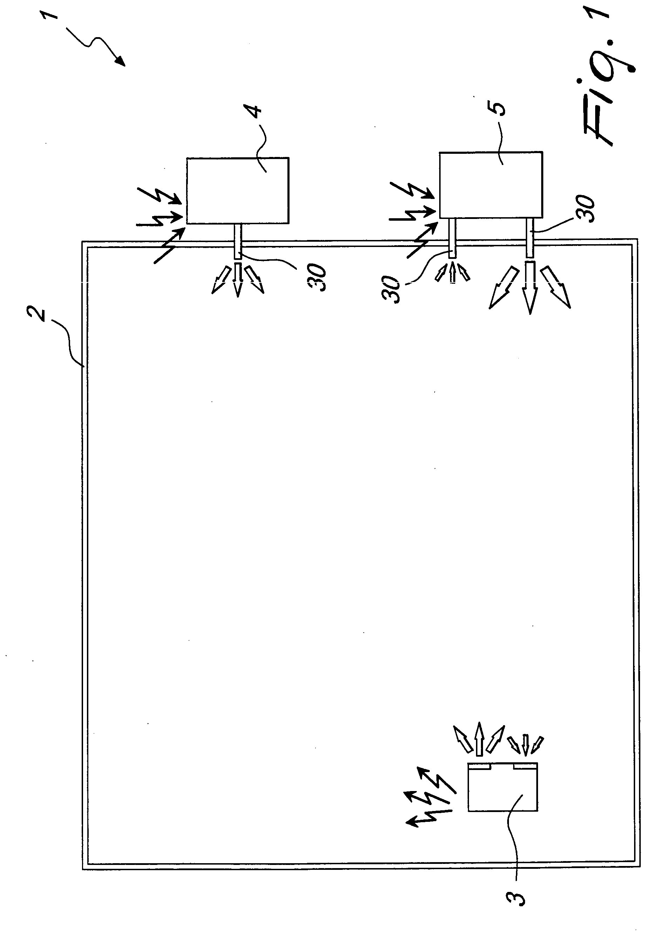

The oxygen enrichment module and the carbon dioxide absorption module are installed outside the room or environment 2 to be served, preferably outside the building that contains the room to be served, and are connected to the environment 2 by means of appropriate input and output piping 30, as shown schematically in Figure 1.

The system allows to choose between at least three operating procedures:
activating only the filtration unit 3 to suppress only particles (minimal operation of the system);
activating also the UV lamps for the sterilization of the air (operation which allows to limit the probability of transmission of contagion within the protected environment);
activating also the oxygen generation module to also produce the accelerating effect on healing processes in case of disease.

The activation policies can be of various kinds and can be programmed in a manner similar to the way in which an ordinary home heating system is programmed.

The wired transmission system is preferably based on the CAN® bus transmission system developed by Bosch® and widely used in the automotive field due to its exceptional features of robustness, immunity to noise, practicality and low cost.

The wireless transmission system instead is based on the ZigBee® protocol, which is a new protocol studied specifically for peer-to-peer wireless networks with a low flow of transmitted data, which is also characterized by exceptional robustness against noise, extremely low energy consumption and good transmission-reception distance.

The great advantages of this architecture are the extremely low desaturation energy required by the material, in comparison to the PSA and/or TSA cycle of a carbon dioxide adsorber using carbon or solid or liquid diethanolamine; the practically nil desaturation inertia and the extraordinary absorption capacity of this new material, which allows to work even with extremely low carbon dioxide concentrations in the air to be purified.

In practice it has been found that the invention achieves the intended aim and objects, providing an apparatus and a method which are capable of assisting the oxidation and consequent elimination of all pathogens performed by antibodies in performing their ordinary functions. In particular, the antibodies "burn" the agents recognized as harmful with the aid of the oxygen.

The controlled increase in the amount of oxygen within rooms populated by human beings is an extremely valid help for immune defenses, which in practice benefit from an effect which accelerates oxidation processes, which consequently become faster and assist healing processes.

Another effect achieved by the invention is to allow to regenerate the air completely by suppressing the carbon dioxide produced by human respiration within the served enclosed spaces.

In this manner, the need for replacing the air with external air can be limited greatly or even eliminated.

This allows to limit greatly the inflow of pollutants into the enclosed spaces, if this is a problem, and to reduce greatly the heating and/or conditioning costs of enclosed spaces both in winter and in summer, which are mainly due to the need to bring the air introduced from outside to a comfortable temperature.

## Claims

1. An apparatus for treating, purifying and reconditioning air within enclosed environments with human presence, said apparatus comprising: filtration means (3) adapted to draw air from an environment (2) and to introduce air deprived of fine particles into said environment (2); said filtration means comprising a module control PLC (9), oxygen enrichment means (4) adapted to draw air from the outside and to introduce air enriched with oxygen into said environment (2); carbon dioxide adsorbent means (5) adapted to draw air from said environment (2) and to introduce air with a reduced content of carbon dioxide into said environment (2); said filtration means comprising a filtration module (3) having a fan (6) that circulates air through a filter (7) for suppressing fine and ultrafine particles; said filtration module (3) further comprising a differential pressure sensor (10) arranged in the output of said filter (7), and a current sensor (11) detecting the current absorbed by said fan (6); said sensors (10, 11) supplying a signal to said module control PLC (9) that assesses the state of clogging of said filter (7); said carbon dioxide adsorbent means comprising a Temperature Swing Adsorber (23); said carbon dioxide adsorbent means (5) comprising a carbon dioxide sensor (26) and an adsorber control PLC (24); said carbon dioxide sensor (26) supplying a signal to said adsorber control PLC (24) for managing the operation of said Temperature Swing Adsorber (23).

2. The apparatus according to claim 1, **characterized in that** said filter (7) is of the HEPA type.

3. The apparatus according to claim 1, **characterized in that** said filtration module (3) comprises UV lamps (8) adapted to kill the bacteria and/or microbes in output from said module (3).

4. The apparatus according to claim 1, **characterized in that** said filtration module (3) comprises an oxygen sensor arranged downstream of said filter (7) and suitable to detect the percentage of oxygen that is present in the air passing through said filter (7).

5. The apparatus according to claim 3, **characterized in that** said filtration module (3) comprises a current sensor (12) for measuring the current absorbed by said UV lamps (8) or a sensor for measuring the brightness of said lamps (8) allowing said module control PLC (9) to know and to report to the utilizer whether the lamps (8) have to be replaced or continue to operate correctly.

6. The apparatus according to claim 1, **characterized in that** said filtration module (3) comprises a sensor for detecting dispersed airborne particles, which is arranged within the module (3) before the filtration chain of said module (3), allowing the control PLC (9) to power-on/off said filtration module (3) according to the quantity of suspended particles detected in said environment (2).

7. The apparatus according to one or more of the preceding claims, **characterized in that** said oxygen enrichment means comprises an oxygen enrichment module (4) comprising an oxygen generator (16), an oxygen control PLC (17) for controlling the oxygen generator, and an oxygen sensor (19) generating a signal which is used by said oxygen control PLC (17) to manage the operation of said oxygen generator (16);
said oxygen generator being of the Pressure Swing Adsorber (PSA) type.

8. The apparatus according to claim 1, **characterized in that** said PSA has an active element constituted by zeolites.

9. The apparatus according to claim 1, **characterized in that** said Temperature Swing Adsorber (23) has an active element constituted by zeolites.

10. The apparatus according to one or more of the preceding claims, **characterized in that** it comprises a system (18) for communication between said modules (3, 4, 5) which can be either of the radio type for wireless transmissions and of the wired type with a field bus.

11. The apparatus according to one or more of the preceding claims, **characterized in that** said filtration module (3) is a stand-alone cabinet; said oxygen enrichment module (4) and said carbon dioxide absorption module (5) being installed outside said environment (2) and being connected to said environment (2) by means of input and output piping (30).

## Patentansprüche

1. Eine Vorrichtung zur Behandlung, Reinigung und Rekonditionierung von Luft in geschlossenen Umgebungen in denen Menschen anwesend sind, wobei die Vorrichtung Folgendes umfasst:
Filtriermittel (3) geeignet Luft aus einem Raum (2) abzuziehen und Luft ohne feine Partikel in den Raum (2) einzuführen; wobei die Filtriermittel eine Modulsteuerungs-SPS (9) umfassen, Sauerstoffanreicherungsmittel (4) geeignet Luft von außen anzuziehen und mit sauerstoffangereicherte Luft in den Raum (2) einzuführen; Kohlendioxidadsorbiermittel (5) geeignet Luft aus der Umgebung (2) abzuziehen und Luft mit einem reduzierten Kohlendioxidgehalt in den Raum (2) einzuführen; wobei die Filtriermittel ein Filtriermodul (3) mit einem Ventilator (6) umfassen, der Luft durch einen Filter (7) zirkulieren lässt, um feine und ultrafeine Partikel zu unterdrücken; wobei das Filtriermodul (3) weiter einen Differenzdrucksensor (10) umfasst, der am Ausgang des Filters (7) angeordnet ist und einen Stromsensor (11) der den von dem Ventilator (6) verbrauchten Strom erfasst; wobei die Sensoren (10, 11) ein Signal an die Modulsteuerungs-SPS (9) senden, die den Verstopfungszustand des Filters (7) erfasst; wobei die Kohlendioxidadsorbiermittel (5) einen Temperature Swing Adsorber (23) umfassen, wobei die Kohlendioxidadsorbiermittel (5) einen Kohlendioxidsensor (26) und eine Adsorbersteurerungs-SPS (24) umfassen; wobei der Kohlendioxidsensor (26) ein Signal an die Adsorbersteurerungs-SPS (24) sendet, um den Betrieb des Temperature Swing Adsorbers (23) zu steuern.

2. Die Vorrichtung gemäß Anspruch 1, **dadurch gekennzeichnet, dass** der Filter (7) vom HEPA-Typ ist.

3. Die Vorrichtung gemäß Anspruch 1, **dadurch gekennzeichnet, dass** das Filtriermodul (3) UV-Lampen (8) umfasst, die ausgebildet sind, um die Bakterien und/oder Mikroben zu töten, die aus dem Modul (3) kommen.

4. Die Vorrichtung gemäß Anspruch 1, **dadurch gekennzeichnet, dass** das Filtriermodul (3) einen Sauerstoffsensor umfasst, der stromabwärts von dem Filter (7) angeordnet und geeignet ist, den Prozentsatz an Sauerstoff zu erfassen, der in der Luft die durch den Filter (7) dringt vorhanden ist.

5. Die Vorrichtung gemäß Anspruch 3, **dadurch gekennzeichnet, dass** das Filtrationsmodul (3) einen Stromsensor (12) zum Messen des Stroms der von den UV-Lampen (8) verbraucht wird oder einen Sensor zur Messung der Helligkeit der Lampen (8) umfasst, der es der Modulsteuerungs-SPS (9) ermöglicht zu wissen und dem Nutzer mitzuteilen, ob die Lampen (8) ausgetauscht werden müssen oder weiter korrekt arbeiten.

6. Die Vorrichtung gemäß Anspruch 1, **dadurch gekennzeichnet, dass** das Filtrationsmodul (3) einen Sensor zur Erfassung dispergierter in der Luft schwebender Partikel umfasst, der vor der Filtrationskette des Moduls (3) in dem Modul (3) angeordnet ist, wodurch es der Steuerungs-SPS (9) ermöglicht wird, das Filtrationsmodul (3) ein- oder auszuschalten je nach der Menge suspendierter Partikel, die in dem Raum (2) erfasst wird.

7. Die Vorrichtung gemäß einem oder mehreren der obigen Ansprüche, **dadurch gekennzeichnet, dass** das Sauerstoffanreicherungsmittel ein Sauerstoffanreicherungsmodul (4) umfasst, dass einen Sauerstofferzeuger (16), eine Sauerstoffsteuerungs-SPS (17) zur Steuerung des Sauerstofferzeugers und einen Sauerstoffsensor (19) umfasst, der ein Signal erzeugt, welches von der Sauerstoffsteuerungs-SPS (17) genutzt wird, um den Betrieb des Sauerstofferzeugers zu steuern, wobei der Sauerstofferzeuger vom Typ eines Druckwechseladsorbers, Pressure Swing Adsorber - PSA -, ist.

8. Die Vorrichtung gemäß Anspruch 1, **dadurch gekennzeichnet, dass** der PSA ein aktives Element hat, das aus Zeolithen besteht.

9. Die Vorrichtung gemäß Anspruch 1, **dadurch gekennzeichnet, dass** der Temperature Swing Adsorber (23) ein aktives Element hat, das aus Zeolithen besteht.

10. Die Vorrichtung gemäß einem oder mehreren der obigen Ansprüche, **dadurch gekennzeichnet, dass** sie ein System (18) zur Kommunikation zwischen den Modulen (3, 4, 5) umfasst, dass entweder vom Funktyp für drahtlose Übertragungen oder vom verdrahteten Typ mit einem Feldbus sein kann.

11. Die Vorrichtung gemäß einem oder mehreren der obigen Ansprüche, **dadurch gekennzeichnet, dass** das Filtrationsmodul (3) ein freistehender Schrank ist, wobei das Sauerstoffanreicherungsmodul (4) und das Kohlendioxidadsorbtionsmodul (5) außerhalb des Raums (2) installiert und mit dem Raum (2) durch eine Eingangs- und Ausgangs-Rohrverbindung (30) verbunden sind.

## Revendications

1. Appareil pour le traitement, la purification et le reconditionnement d'air dans des environnements clos avec présence humaine, ledit appareil comprenant:
un moyen de filtration (3) adapté pour aspirer de l'air à partir d'un environnement (2) et pour introduire de l'air dénué de particules fines dans ledit environnement (2); ledit moyen de filtration comprenant un PLC de commande de module (9), un moyen d'enrichissement en oxygène (4) adapté pour aspirer de l'air depuis l'extérieur et pour introduire de l'air enrichi en oxygène dans ledit environnement (2); un moyen d'adsorption de dioxyde de carbone (5) adapté pour aspirer de l'air à partir de l'environnement (2) et pour introduire de l'air avec une teneur réduite en dioxyde de carbone dans ledit environnement (2); ledit moyen de filtration comprenant un module de filtration (3) comportant un ventilateur (6) chargé de faire circuler de l'air à travers un filtre (7) pour supprimer les particules fines et ultrafines; ledit module de filtration (3) comprenant en outre un capteur de pression différentielle (10) disposé dans la sortie dudit filtre (7), et un capteur de courant (11) détectant le courant absorbé par ledit ventilateur (6); lesdits capteurs (10, 11) fournissant un signal audit PLC de commande de module (9), lequel évalue l'état de colmatage dudit filtre (7); ledit moyen d'adsorption de dioxyde de carbone comprenant un adsorbeur à température alternée (23); ledit moyen d'adsorption de dioxyde de carbone (5) comprenant un capteur de dioxyde de carbone (26) et un PLC de commande d'adsorbeur (24); ledit capteur de dioxyde de carbone (26) fournissant un signal audit PLC de commande d'adsorbeur (24) pour gérer le fonctionnement dudit adsorbeur à température alternée (23).

2. Appareil selon la revendication 1, **caractérisé en ce que** ledit filtre (7) est de type HEPA.

3. Appareil selon la revendication 1, **caractérisé en ce que** ledit module de filtration (3) comprend des lampes UV (8) adaptées pour tuer les bactéries et/ou les microbes dans la sortie dudit module (3).

4. Appareil selon la revendication 1, **caractérisé en ce que** ledit module de filtration (3) comprend un capteur d'oxygène disposé en aval dudit filtre (7) et approprié pour détecter le taux d'oxygène présent dans l'air traversant ledit filtre (7) .

5. Appareil selon la revendication 3, **caractérisé en ce que** ledit module de filtration (3) comprend un capteur de courant (12) destiné à mesurer le courant absorbé par lesdites lampes UV (8) ou un capteur destiné à mesurer la luminosité desdites lampes (8), permettant audit PLC de commande de module (9) de savoir et d'indiquer à l'utilisateur si les lampes (8) doivent être remplacées ou continuent de fonctionner correctement.

6. Appareil selon la revendication 1, **caractérisé en ce que** ledit module de filtration (3) comprend un capteur destiné à détecter des particules dispersées en suspension dans l'air, lequel est disposé à l'intérieur du module (3) en amont de la chaîne de filtration dudit module (3), permettant au PLC de commande (9) d'allumer/éteindre ledit module de filtration (3) en fonction de la quantité de particules en suspension détectée dans ledit environnement (2).

7. Appareil selon l'une ou plusieurs des revendications précédentes, **caractérisé en ce que** ledit moyen d'enrichissement en oxygène comprend un module d'enrichissement en oxygène (4) comprenant un générateur d'oxygène (16), un PLC de commande d'oxygène (17) destiné à commander le générateur d'oxygène, et un capteur d'oxygène (19) générant un signal utilisé par ledit PLC de commande d'oxygène (17) pour gérer le fonctionnement dudit générateur d'oxygène (16); ledit générateur d'oxygène étant de type adsorbeur à pression alternée (PSA).

8. Appareil selon la revendication 1, **caractérisé en ce que** ledit PSA comporte un élément actif constitué de zéolites.

9. Appareil selon la revendication 1, **caractérisé en ce que** ledit adsorbeur à température alternée (23) comporte un élément actif constitué de zéolites.

10. Appareil selon l'une ou plusieurs des revendications précédentes, **caractérisé en ce qu'**il comprend un système (18) permettant de communiquer entre lesdits modules (3, 4, 5), lequel peut être soit de type radio pour des transmissions sans fil, soit de type câblé avec un bus de champ.

11. Appareil selon l'une ou plusieurs des revendications précédentes, **caractérisé en ce que** ledit module de filtration (3) est une armoire indépendante; ledit module d'enrichissement en oxygène (4) et ledit module d'adsorption de dioxyde de carbone (5) étant installés à l'extérieur dudit environnement (2) et raccordés audit environnement (2) par une canalisation d'entrée et de sortie (30).
